# EUROPEAN PATENT SPECIFICATION

(11) **EP 3 796 974 B1**
(45) Date of publication and mention of the grant of the patent: **08.01.2025**
(21) Application number: 19730959.4
(22) Date of filing: 20.05.2019
(51) Int. Cl.: A61P 1/02, A61Q 11/00, A61K 9/00, A61K 36/064

(54) **A TOPICAL, ORAL, PHARMACEUTICAL COMPOSITION FOR USE IN THE TREATMENT OF INFLAMMATION-BASED DISEASES OF THE ORAL CAVITY**
TOPISCHE, ORALE, PHARMAZEUTISCHE ZUSAMMENSETZUNG ZUR VERWENDUNG BEI DER BEHANDLUNG VON ENTZÜNDUNGSBEDINGTEN KRANKHEITEN DER MUNDHÖHLE
COMPOSITION PHARMACEUTIQUE TOPIQUE, ORALE, DESTINÉE À ÊTRE UTILISÉE DANS LE TRAITEMENT DE MALADIES À BASE D'INFLAMMATION DE LA CAVITÉ BUCCALE

(30) Priority: 21.05.2018 IT 201800005538
(43) Date of publication of application: 31.03.2021
(73) Proprietor: Stefani, Roberto, 34123 Trieste (IT)
(72) Inventor: Stefani, Roberto, 34123 Trieste (IT)
(74) Representative: Emmi, Mario
(86) International application number: PCT/IB2019/054127
(87) International publication number: WO 2019/224683

(56) References cited:
- COLIN HILL ET AL: "The International Scientific Association for Probiotics and Prebiotics consensus statement on the scope and appropriate use of the term probiotic : Expert consensus document", NATURE REVIEWS GASTROENTEROLOGY, vol. 11, no. 8, 1 August 2014 (2014-08-01), London, pages 506 - 514, XP055447207, ISSN: 1759-5045, DOI: 10.1038/nrgastro.2014.66
- ULLAH MATI ET AL: "Snapshot of the Probiotic Potential of Kluveromyces marxianus DMKU-1042 Using a Comparative Probiogenomics Approach", FOODS, vol. 12, no. 23, 30 November 2023 (2023-11-30), CH, pages 4329, XP093187365, ISSN: 2304-8158, DOI: 10.3390/foods12234329
- ANONYMOUS: "BIOSYMPA: PROBIOTICO UNICO E INNOVATIVO", 10 February 2012 (2012-02-10), XP055617898, Retrieved from the Internet <URL:https://www.newsfood.com/biosympa-probiotico-unico-e-innovativo/?pdf=92549> [retrieved on 20190903]
- TECHNOLOGIC PARK ET AL: "PROBIOTIC LACTIC YEAST, Kluveromyces B0399", 10 July 2017 (2017-07-10), XP055617938, Retrieved from the Internet <URL:https://turval.com/products/humans/probiotic-lactic-yeast/170615-brochure-km-b0399-ab.pdf> [retrieved on 20190903]
- MICHAEL MUELLER ET AL: "Production of Xylitol by the Thermotolerant Kluyveromyces marxianus IMB Strains", JOURNAL OF BIOPROCESSING & BIOTECHNIQUES, vol. 01, no. 02, 1 January 2011 (2011-01-01), XP055618023, DOI: 10.4172/2155-9821.1000102e
- EUNJOO PARK ET AL: "Xylitol, an Anticaries Agent, Exhibits Potent Inhibition of Inflammatory Responses in Human THP-1-Derived Macrophages Infected With Porphyromonas gingivalis", JOURNAL OF PERIODONTOLOGY., vol. 85, no. 6, 1 June 2014 (2014-06-01), US, pages e212 - e223, XP055618019, ISSN: 0022-3492, DOI: 10.1902/jop.2014.130455
- S. MACCAFERRI ET AL: "Potential Probiotic Kluyveromyces marxianus B0399 Modulates the Immune Response in Caco-2 Cells and Peripheral Blood Mononuclear Cells and Impacts the Human Gut Microbiota in an In Vitro Colonic Model System", APPLIED AND ENVIRONMENTAL MICROBIOLOGY, vol. 78, no. 4, 9 December 2011 (2011-12-09), US, pages 956 - 964, XP055372581, ISSN: 0099-2240, DOI: 10.1128/AEM.06385-11
- SARA QUARELLA ET AL: "Draft Genome Sequence of the Probiotic Yeast Kluyveromyces marxianus fragilis B0399", GENOME ANNOUNCEMENTS, vol. 4, no. 5, 27 October 2016 (2016-10-27), XP055539818, DOI: 10.1128/genomeA.00923-16

## Description

### Technical Field

The present invention relates to a topical, oral, pharmaceutical composition for use in the treatment of diseases of the oral cavity, in particular inflammation-based diseases involving mucosae and supporting structures of teeth and implants, such as, for example, gingivitis, periodontitis, peri-implant mucositis and peri-implantitis, wherein the active ingredient consists of at least one probiotic (i.e. alive and viable) yeast.

In particular, the present invention relates to a topical, oral, pharmaceutical composition for use in the above-mentioned treatment, wherein said active ingredient consists of at least one probiotic (i.e. alive and viable) yeast capable of crossing the gastric barrier (i.e. gastric juices in the stomach and bile) undamaged and reaching the gut in alive and viable form.

More specifically, the present invention relates to a topical, oral, pharmaceutical composition for use in the above-mentioned treatment, wherein the active ingredient consists of at least the yeast *Kluyveromyces marxianus fragilis* B0399.

### Background Art

The diseases of the oral cavity that are of interest to the present invention are well-known inflammation-based diseases such as, for example, gingivitis, periodontitis, peri-implant mucositis and peri-implantitis.

Local inflammation is triggered by accumulation of gram-negative pathogenic bacteria (that are present in the plaque), which alter the composition of the normal oral microbiota (thus causing various forms of dysbiosis). The pathologic action of such bacteria is fostered and modulated, in different persons, by inflammation-promoting systemic factors. It can therefore be stated that local inflammation and systemic inflammation are bound by a bidirectional relationship. In this respect, it is worth reminding that, according to the scientific literature, all inflammatory forms involving oral tissues, and in particular all forms of periodontitis that lead to destruction of tooth supporting structures, allow for systemic absorption of gram-negative pathogenic bacteria, which therefore represent an important risk factor for numerous chronic systemic inflammatory diseases. Merely by way of example, these include arteriosclerosis with cerebrovascular accidents (doubled risk), coronary diseases (doubled risk), respiratory diseases (2- to 5-fold risk), pregnancy complications (4-to 7-fold risk), diabetes (2- to 4-fold risk), and so on. The periodontal disease is a disease that cannot be healed, and the therapy that is traditionally used is mainly aimed at countering and limiting it.

The conventional therapy uses consolidated, tested and validated surgical and/or non-surgical hygienic-mechanic protocols. These are mainly based on the elimination of the triggering factor, i.e. the periodontal pathogenic gram-negative bacteria. The cornerstones of this therapy are, generally, good daily hygiene by the patient, associated with a non-surgical out-patient periodontal therapy at predefined intervals. In the most difficult and serious cases, a surgical periodontal therapy is also used, which is aimed at remodelling and/or rebuilding the anatomic components that have been seriously harmed by the disease.

Such conventional treatments also make use of antibiotics, disinfectants, antiseptics and/or antimicrobial agents such as, for example, chlorhexidine. However, such agents can only control, but not resolve, the disease, and may also have a number of side effects (anaphylactic reactions, transverse resistances, tooth pigmentation, and so on).

Recent approaches described in the scientific literature [e.g. **1, 2**] and in the patent literature [e.g. **3**] propose the use of probiotics in the oral cavity.

For clarity's sake, it is necessary to remind beforehand that, when it comes to "*probiotics"*, reference is made to microorganisms, bacteria or yeasts that are taken "*alive and viable"*, so that they can reach the reference mucosa and "adhere thereto and multiply" to perform their different immune and metabolic functions over time. "*Alive and viable"* represents the fundamental characteristic inherent in a probiotic microorganism, which must be able to attach to mucosae and multiply thereon in order to replicate its functions over time. A dead yeast, or a part thereof, or a derivative thereof, since it cannot regenerate itself, can only perform a temporary administration-related activity.

It must also be said that "probiotic" is a definition that appears on circulars by the Italian Ministry, which in turn is the translation of a definition recognized at worldwide level ("*microrganismi vivi e vitali che conferiscono benefici alla salute dell'ospite quando consumati in adequate quantità come parte di un alimento o di un integratore"* ["*alive and viable microorganisms that, when assumed in adequate amounts as part of a food or food supplement, confer a health benefit to the host"*]: http://www.salute.gov.it/portale/temi/p2 6.jsp?id=1426&ar ea=Alimenti%20particolari%20e%20integratori&menu=integrat ori). In brief, using probiotics (which are alive and viable) means providing the environment (organism or tissue) with functional elements capable of integrating their own functions according to requests from the environment itself, thus contributing to creating a symbiotic balance, the efficiency level of which defines the actual border between health and disease. These concepts have been recognized in the scientific literature for more than a hundred years, but are still unclear within the scope of a conventional medical approach to health problems.
**1**- In Cannon M., et al., Effectiveness of CRT at measuring the salivary level of bacteria in caries prone children with probiotic therapy, J Clin Pediatr Dent 2013;38(1) :55-60*,* 60 children took probiotic bacteria in the form of tablets for 30/50 days. A reduction in the number of cariogenic bacteria at salivary level was observed.
***2*** - In Flichy-Fernández A.J., et al., **The effect of orally administered probiotic Lactobacillus reuteri-containing tablets in peri-implant mucositis: a double-blind randomized controlled trial,** *J Periodont Res 2015 Feb 25,* 34 patients with 77 implants affected by mucositis were treated with tablets of probiotic bacteria for 30 days. A reduction in pro-inflammatory cytokines was obtained.
***3*** - EP 1 852 122 A1 describes the use of *Enterococcus faecium* and/or *Saccharomyces boulardii* to alleviate the symptoms of gingivitis, periodontitis, halitosis. The bacterium *Enterococcus faecium* is not a probiotic. *Saccaromyces boulardii* is a probiotic yeast, but belongs to a yeast family other than *genus Kluyveromyces* (in particular, it is different from *Kluyveromyces marxianus fragilis* B0399), and therefore the functions performed may be similar in some aspects, but not equal (see, for example, **7**). In particular:
   - B0399 is much more represented in Caucasian populations because it comes from goat milk (kefir), which has always and usually been consumed. *S. boulardii* is a rare yeast, in that it is derived from a rare plant, lychee, which can only be found in certain regions of the world (Indochina).
   - There are significant differences between B0399 and *S. boulardii* as regards expressed functional performances. B0399 has generally proven much more efficient and versatile (see, for example, **7**).
   Undoubtedly, it appears that *S. boulardii* survives within an aqueous gel, such as the one used for toothpaste production; certainly, *Enterococcus faecium* does not survive. The patent describes a topical use thereof on gums by rubbing a powder applied on a toothbrush. Only surface observation could lead to the conclusion that this is a toothpaste. Actually, only the gesture is similar, but no toothpaste is used (which cannot be created by using enterococcus). Moreover, no mention is made regarding the use of masks or the introduction of the active principle into gum pockets. The action of the active principle in the masks and in the gum pockets is more effective when the principle is kept in close contact with the pathogens, protected from washing salivary fluids and from stirring movements made by the tongue and the cheeks (changed environment/changed effect biology).
**4** - US 4,980,151 A describes a yeast, comprised in the *genus Kluyveromyces,* as an anti-periodontitis agent. It can be prepared, for example, as a toothpaste, a collutorium, a chewing gum.
**5** - FR 2999 085 A1 also describes a yeast as an active agent for protecting the oral mucosa. *Kluyveromyces marxianus* is described as one possible therapeutic agent. However, in both documents **4** and **5** the yeasts employed are dead, as opposed to alive and viable, and therefore they are not probiotic. In fact, mixtures of yeast extracts are proposed, which are extracted and filtered (and therefore are no longer alive and viable, i.e. they are non-probiotic) for use in the mouth. The action of such filtrates is not the same as that exerted by alive yeast cells, which adhere to mucosae and replicate (see **7**). Such action, which is clearly non-probiotic, is recognized as a *pre-biotic* action, i.e. an action supported by substances belonging to a probiotic structure, but lacking the viable cellular element they are part of. In the literature, the pre-biotic action is recognized, but is kept well distinct and considered different from the probiotic one.
**6** - WO 2008/155120 A2 describes the use of bacteria and yeasts, which are declared as being "non-pathogenic" and "non-invasive", in the treatment of oral mucositis.

Such definitions do not imply that such microorganisms are probiotics.

In addition, even assuming that such microorganisms are probiotics, they are "genetically modified" for the purpose of enhancing specific performance characters thereof, and this treatment, since it heavily modifies the genetic equipment of the microorganism, makes it different from the previous one.

For this reason, at this point, even assuming that it is still alive (which is uncertain and unlikely), it will be completely different from the initial microorganism. The document includes a long list of bacteria and yeasts that are modified in many ways and administered also by parenteral administration, intravenous injection, with antibiotics, in liquid carriers, but all of these conditions have nothing to share with a possible use of probiotics and give clearly to understand that these are not alive and viable microorganisms, but parts (peptides) of dead microorganisms.

**7** - Ida M. Smith et al.: Kluyveromyces marxianus and Saccharomyces boulardii Induce Distinct Levels of Dendritic Cell Cytokine Secretion and Significantly Different T Cell Responses In Vitro, PLOS ONE, vol. 11, no. 11, 29 November 2016 (2016-11-29), page e0167410, XP055539455, DOI: 10.1371/journal.pone.0167410, explains how the alive yeasts taken into consideration can affect the immune system of man, and that such beneficial action is more efficient and complete when exerted by an alive microorganism rather than an extract obtained from (dead) parts of the same microorganisms.6

### Shortcomings of the Prior Art

Conventional treatments, as well as the latest ones mentioned above, suffer all from some drawbacks, such as, for example:
- Conventional protocols do not consider the host's inflammatory response (promoting factor) to be important in modulating the aggressiveness of the bacterial pathogen that is the factor that triggers the disease locally. This is an important limitation from a therapeutic viewpoint and also as far as prevention is concerned.
- Only EP 1 852 122 A1 describes the possibility of a dual local and systemic action of the probiotics employed, considering such two actions as separate and hence the possibility of choosing either one of them. Actually, the two actions, local and systemic, are synergical and mutually implemented, and must therefore occur simultaneously. In all other cases, the probiotics taken into account are used only topically at oral level. This limitation probably comes from the fact that the bacterial probiotics used, once they have arrived in the oral cavity, either cannot cross the gastric barrier undamaged, remaining alive and viable, or cannot cross it in an adequate, effective amount, thus not exerting their beneficial probiotic action at the level of the intestinal mucosa.
- In no cases the innovative approaches described in the scientific and patent literature (1, 2, 3) envisage the use of probiotics to be directly introduced, e.g. by means of a syringe or a cannula, into the periodontal and peri-implant pockets, or positioned and maintained on teeth and gums by means of suitable medical devices (e.g. masks) built *ad hoc.*

As a consequence, a need is still felt for solving the technical problem of reducing the (local) inflammation of oral mucosae and tooth/implant supporting structures and also the (systemic) inflammation at gut level, simultaneously re-balancing the bacterial flora (microbiota) of both the mouth and the gut by means of the same active principle.

### Technical Problem

Therefore, industry operators still strongly demand for the availability of a pharmaceutical composition capable of topically treating the inflammatory states of oral mucosae and tooth/implant supporting structures and the diseases related thereto or derived therefrom, such as, for example, gingivitis, periodontitis, peri-implant mucositis and peri-implantitis, while also at the same time significantly reducing the inflammatory state of the gut, re-balancing the microbiota thereof, and thus ameliorating the systemic inflammatory state (overall health condition) of the individual.

It is the object of the present invention to provide an adequate solution to the above-described technical problem.

### Summary of the Invention

The present inventor has now found that a topical, oral, pharmaceutical composition in which the active ingredient consists of at least one suitable probiotic (eubiotic) yeast, i.e. alive and viable, capable of crossing the gastric barrier undamaged and reach the gut in alive and viable form, can provide an adequate (local/systemic) solution to the technical problem highlighted above.

It is therefore an object of the present invention to provide a topical, oral, pharmaceutical composition as defined above for use in the treatment of inflammation-based diseases of the oral cavity, as set out in the appended independent claim.

Some preferred embodiments of the present invention are set out in the appended dependent claims.

The preferred embodiments of the present invention illustrated in the following description only have an explanatory purpose and are by no means intended to limit the application scope of the present invention, which will be immediately apparent to those skilled in the art.

### Detailed Description of the Invention

The present invention relates to a topical, oral, pharmaceutical composition for use in the treatment of inflammation-based diseases of the oral cavity, wherein said diseases are selected from the group consisting of gingivitis, periodontitis, peri-implant mucositis and peri-implantitis, and wherein the active ingredient consists of an effective amount of at least one probiotic yeast as defined in the claims, alive and viable, which can cross the gastric barrier undamaged and reach the gut in alive and viable form.

In said composition, said at least one probiotic yeast is present in an amount at least equal to 10 million cfu/dose (10⁷ cfu/dose); preferably, ≥ 10⁷ cfu/dose; more preferably, ≥ 2•10⁷ cfu/dose; even more preferably, ≥ 5•10⁷ cfu/dose; even more preferably, ≥ 10⁸ cfu/dose; even more preferably, ≥ 10⁹ cfu/dose; for example, in the range of 2•10⁷ cfu/dose to 10⁹ cfu/dose.

In general, yeasts are a small part (1%) of the so-called oral and intestinal microbiota, while the remaining 99% is represented by bacteria, mainly Lactic and Bifid ones. Yeasts contribute, e.g. when associated with Lactic bacteria, to exerting a general defense and support action (microbiota biodiversity). Therefore, yeast intake is generally important for keeping the microbiota in good efficiency conditions (territorial resilience). Merely by way of example, many articles in the literature magnify the positive effects of the yeast *Saccaromyces boulardii* both generally on the microbiota and specifically in the individual diseases involving the gut.

For the purposes of the present invention, the active ingredient consists of at the yeast is *Kluyveromyces marxianus fragilis* B0399.

This strain (produced, for example, by Laboratori Turval Italia, S.r.l., Udine) was typified and filed at the BCCM-Belgian Coordinated Collections of Microorganisms, Culture Collection Mycoteque de l'Université Catholique de Lovain (Belgium) under code B0399 (April 1999, Access Number 55798). In 2009 it was recognized as being *probiotic* (i.e. capable of ameliorating the balance of the microbial flora) by the Italian Ministry of Health.

This is the only probiotic yeast that is completely "natural" and "gut friendly", and is naturally found in food products (e.g. kefir from goat milk) and in the human intestine.

*Kluyveromyces marxianus fragilis* B0399 effectively performs many important functions, such as, for example: stimulating the growth and efficiency of an individual's beneficial endogenous bacterial flora (bifidobacteria); exerting an antimycotic action against Candida; fighting pathogens by lowering the pH; reinforcing the coating epithelia by adhering to the surfaces and taking nutrients away from them. Also of fundamental importance is its capability of producing short-chain fatty acids (SCFA), which are substances used by tissue cells in their energy production (Krebs) cycle to achieve better functional efficiency. Being active at low doses, B0399 crosses the gastric barrier undamaged, resists antibiotics, digests lactose and, most importantly, modulates the host's immune system by re-balancing the levels of cytokines and providing immunostimulating substances such as β-glucanes and oligosaccharides.

Such an active principle offers the operator several possibilities of use:
- it has proven to effectively stimulate the development of intestinal microflora (in particular, bifidobacteria) even at low doses, e.g. 100 times less than *S. boulardii,* Lactobacilli and Bifidobacteria;
- it crosses the gastric barrier undamaged and reaches the gut still alive and viable;
- it colonizes the gut effectively;
- it has antimycotic activity, particularly against Candida Albicans;
- it competes with pathogens by a) adhering to the epithelium and preventing other microorganisms from settling thereon, b) using nutrients and taking them away from pathogens, c) stimulating mucosal trophism in several manners, d) lowering the ambient pH;
- it modulates the immune response by re-balancing cytokines and the production of immunostimulants such as β-glucanes and oligosaccharides (GOS, FOS, MOS);
- it exerts a beneficial action on IBS (irritable bowel syndrome);
- it digests lactose, due to high production of β-galactosidase;
- it is naturally resistant to antibiotics and, unlike bacteria, has no mechanisms of antibiotic resistance;
- furthermore, significant production of acetate and proprionate has been observed *in vitro,* which confer energy on intestinal cells, thereby promoting all functions performed by them.

As a result, B0399 has proven to be:
- effective in countering inflammatory intestinal diseases;
- flexible to use, since it allows the creation of different solutions, whether alone or with the addition of other microorganisms or active principles, which may be administered in different ways; this makes it possible to define wholly customized therapeutic protocols for a specific individual;
- non-competitive with conventional therapeutic protocols; on the contrary, it amplifies the effect thereof, and can be repeated multiple times or used for long periods.

When compared with lactic bacilli *Lactobacillus* ssp., B0399 has properties and indications that imply greater efficiency:
- it easily crosses, in a large amount, the gastric barrier;
- it produces a greater quantity of substances that are useful for the microbiota, such as, for example, oligosaccharides (MOS, FOS, GOS), β-glucanes, galactosidase, insulinase, glucanase;
- it resists microbial agents;
- it is specific for lactose-intolerant individuals;
- it is insensitive to antibiotics;
- it produces a large amount of energy for the cell in the form of ATP;
- it has very low efficacious doses;
- it performs a specific anti-Candida action.

When compared with the yeast *Saccaromyces boulardii,* B0399 has properties and indications that imply greater efficiency:
- it has β-galactosidase activity;
- it produces lactic acid and acidifies the ambient pH to the detriment of pathogens;
- it has no contraindications;
- it is effective in stimulating the performance of Bifidobacteria (butyric acid);
- it exerts an antimycotic action;
- it can normally be found in foods;
- it is efficacious at low doses (millions, as opposed to billions, of CFUs/dose).

Further advantageous characteristics
- Formation of useful compounds
- In anaerobic conditions, it produces twice as much lactic acid compared to heterofermentants, and produces no CO₂. Therefore, it digests lactose thanks to its good capability of producing the β-galactosidase enzyme (which is very good for lactose-intolerant individuals).
- It can induce production of β-glucanes and oligosaccharides (GOS, FOS, MOS) through β-glucanase and insulinase. Therefore, it also exerts a prebiotic action, in addition to the already known probiotic action. It follows that it can also be defined as a "symbiotic" microorganism.

It is recommended by the producer (Turval) in cases of abdominal aerophagia, gut dismicrobism, IBS, dermatitis and Candida albicans.

To the inventor's knowledge, B0399, the beneficial effects of which at gut level are known, has until now been neither described nor used directly (i.e. topically) on mucosae and tissues of the oral cavity.

As already anticipated, the active ingredient of the topical, oral, pharmaceutical composition for use in the treatment of inflammation-based diseases of the oral cavity consists of an effective amount of at least the probiotic yeast *Kluyveromyces marxianus fragilis* B0399.

In said composition, said at least one probiotic yeast is present in an amount at least equal to 10 million CFUs per dose (10⁷ cfu/dose); preferably, ≥ 10⁷ cfu/dose; more preferably, ≥ 2•10⁷ cfu/dose; even more preferably, ≥ 5•10⁷ cfu/dose; even more preferably, ≥ 10⁸ cfu/dose; even more preferably, ≥ 10⁹ cfu/dose; for example, in the range of 2•10⁷ cfu/dose to 10⁹ cfu/dose.

Expressing said amount in weight terms, said yeast may be generally present in the composition of the invention in an amount preferably ranging from a minimum of 0.005 g to 5 g per dose, more preferably from 0.008 g to 2.5 g per dose; more preferably from 0.08 g to 0.25 g per dose.

As far as the maximum dose is concerned, due to the absolute atoxicity of the microorganism and to the absence of any known unfavourable side effects, it can be stated that no maximum dose exists or may be conjectured; on the contrary, excessive dosage should reasonably do no harm, since it would ensure better efficiency. In such a case, the unused excess yeast would be eliminated by the body through the normal excretory ways.

In a preferred embodiment of the present invention, the microorganism (i.e. the at least one probiotic yeast of the invention) is provided in dehydrated form as powder obtained by known lyophilization processes and, depending on the different compositions and applications, can be administered in different carrier forms.

The dehydrated microorganism(s) of the invention has (have) the characteristic of reactivating when released in a suitable humid site, such as the oral mucosa or the intestinal mucosa, where colonization occurs to provide re-balancing (or eubiosis) among the various species of residential (autochthonous) or transient (allochthonous) bacterial flora.

In order to ensure appropriate crossing of the gastric barrier in all cases, the dehydrated microorganism(s) of the invention may optionally be microencapsulated to become resistant to gastric juices and/or bile. The optional microencapsulation is preferably effected through the use of well-known microencapsulation techniques commonly used in the industry, which will not be described in detail herein.

The pharmaceutical composition of the present invention for use in the treatment of diseases of the oral cavity as previously described may possibly also contain one or more additional active principles or substances known in the art, acting synergically, or performing an adjuvant or complementary function, with the alive microorganism(s) of the invention.

Said optional additional active principles of the present invention can be selected among many substances recognized by competent bodies as being miscible, e.g. probiotics, nutraceuticals, minerals, vitamins, "botanicals" vegetable preparations, prebiotics, enzymes, algae, and any other substances symbiotically compatible with the reference active principle (the yeast).

Merely by way of non-limiting example, said additional substances may be selected from:
- vegetable extracts (rosa canina, acerola, echinacea, ribes nigrum, agrimonia, uncaria, bearberry, grapefruit, calendula, etc.), and/or
- immunostimulating supplements (ginseng, aloe, camu camu, blueberry, propolis, etc.), and/or
- nutraceuticals (limonene, colostrum, flavonoids, etc.), and/or
- vitamins (vitamin C, B-group vitamins, etc.), and/or
- minerals (magnesium, zinc, etc.), and/or
- fibers and prebiotics (FOS, GOS, inulin, hydroxypropyl methylcellulose, etc.), and/or
- amino acids (phenylalanine, L-carnitine, niacin, etc.), and/or
- enzymes, and/or
- flavourings (apricot, elder, orange, etc.), and/or
- essential oils (bergamot, camomile, lemon, peppermint, sage, cinnamon, myrrh, rosemary, melaleuca, etc.), and/or
- anti-agglomerants (magnesium stearate, silica, etc.), and/or
- acidity correctors (citric acid), and/or
- sweeteners (sucralose, maltitol, etc.), and/or
- sugars (fructose, glucose, etc.), and/or
- pigments (titanium dioxide, etc.), and/or
- preservatives (parabens), and/or
- humectants (glycerine), and/or
- other microorganisms and probiotics (*S. cerevisiae,* Bifidobacteria, *Lactobacillus* spp., *S. boulardii,* Enterococcus, etc.).

The compositions of the present invention may be for nutraceutical and/or pharmaceutical and/or cosmetic use, and preferably can be produced as a gel toothpaste, a gel for local surface applications, a miscible powder to be directly introduced into the periodontal pockets, or tablets, capsules, microcapsules or pearls, powders in packets or other containers, single-dose bottles, chewing gums, candies, pills or gels, solutions, emulsions in any form, sprays, mouthwash liquids, or any other technological form for oral administration commonly available in the industry.

Once activated in the mouth (as previously described), the principle can then be swallowed, so that it can continue its beneficial action also at gut level.

As a consequence, the compositions of the present invention may be swallowed after topical oral application, as opposed to being expelled by rinsing, since they are fully compatible with the human body. In particular, the characteristic of B0399 of being able to cross the gastric barrier undamaged allows the composition to exert a prolonged action on intestinal mucosae as well.

### Experimental Section

The following experimental section illustrates, merely by way of example and without by no means limiting the broad application potential of the present invention, some particularly preferred embodiments of the present invention. It goes without saying that all possible implementation and application variants of the same are within the grasp of a person skilled in the art, and as such they fall within the protection scope of the invention.

### Example 1

### Example 1 - Swallowable toothpaste in sealed tube: formulation and use

Using a conventional turbo emulsifier, the following substances are mixed, in this order and at room temperature: glycerine (70 to 80%), xanthan gum (1 to 5%), water (5 to 10%), silica (0.1 to 1%), lyophilized *Kluyveromyces marxianus fragilis* B0399 (5 to 10%), flavouring (0.1 to 1%), essential oil of lemon (0.1 to 1%), limonene (0.1 to 1%), citral (0.1 to 1%) in weight relative to the total weight of the composition. After the addition of each ingredient, the mixture is stirred for a time ranging from approx. 10 to 20 minutes until complete dispersion of the ingredient is attained. Once the toothpaste having the desired appearance/dispersion has been obtained, the product is collected into clean, sanitized drums and then packaged into sealed tubes.

The above toothpaste is applied onto the bristles of a toothbrush and interdental cleaning tools, and ensures a correct distribution of the active principle on all mucosae of the oral cavity. Laboratory tests have shown that 1 centimeter of toothpaste (recommended dose) to be spread over the bristles of a toothbrush contains an effective amount of the active principle taken into consideration herein. Daily use, possibly associated with a subsequent deglutition phase, ensures a natural beneficial (anti-inflammatory and curative) action on the involved tissues of the oral cavity and on the intestinal tract as well. Should the person decide to not swallow the product, it is however recommended to not rinse the mouth after use, so as to promote a prolonged local action of the active principle.

### Example 2 - Powder/gel for periodontal masks

A gel, or a powder, containing the active principle (lyophilized *Kluyveromyces marxianus fragilis* B0399 in an amount ranging from 0.6 g to 1.3 g per 100 g of composition), mixed with a suitable quantity of water or enriched with compatible or adjuvant substances, such as to maintain the nutraceutical formulation, such as typified bacteriocin-producing bacterial strains or phytotherapic active principles having beneficial effects that are adequate for the problem to be treated (rosa canina, acerola, aloe, elements having a prebiotic action, etc.), are introduced into suitable dental masks custom-made by the dentist and are applied onto the dental arches so as to keep the product in prolonged contact with the dental structures and the mucosae. This is a very effective solution for serious cases of periodontitis or peri-implantitis. In fact, the active principle, being protected by the mask, will remain for a long time highly concentrated and stably in contact with the structures to be treated.

### Example 3 -

As an alternative, said gel or said powder of Example 2, possibly suitably enhanced and supplemented with other adjuvant additives appropriately selected among those previously described herein, may also be directly introduced into the periodontal pockets, e.g. by means of a syringe fitted with a thin needle/cannula, so as to exert a more immediate and direct action (this application must be made as an out-patient treatment by skilled and trained personnel).

### Example 4 - Chewing gum

A traditional chewing gum is prepared as commonly known in the art, wherein 0.2 g of lyophilized *Kluyveromyces marxianus fragilis* B0399 per gum piece/dose and optionally an adequate amount of additional adjuvant substances (e.g. probiotics, prebiotics, etc.) are added to the mixture of desired ingredients. This type of solution turns out to be particularly effective when treating inflammatory diseases of the oral cavity. In fact, in addition to squeezing the active principle on teeth and gums, mastication also adds an effective mechanical cleansing action (plaque shattering), associated with recirculation of saliva, enriched with active principle, in the interdental spaces.

### Example 5 - Chewable or swallowable tablets

1.5g chewable or swallowable tablets are prepared with methods and equipment commonly employed in the pharmaceutical art, adding 0.15 g of lyophilized *Kluyveromyces marxianus fragilis* B0399 per tablet and, optionally, an adequate amount of additional adjuvant substances (e.g. probiotics, pre-biotics, etc.) to the mixture of desired ingredients. This type of solution may be the most appropriate one for persons who, for any reason, do not want to swallow toothpaste after brushing. Chewable or suckable tablets, similar to candies, will allow the active principle to remain *in situ* (i.e. in the mouth) while ensuring constant dosage and allowing the active principle to reach the gut in alive and viable form via saliva deglutition.

### Advantages of the Invention

The compositions according to the present invention have advantageously proven to be useful in the dentistry field and, in general, in the medicine field for treating inflammatory diseases of the oral cavity; in particular, they have proven to be useful for treating diseases of the oral mucosa, gingivae, and tooth and/or implant supporting tissues.

Advantageously, the compositions according to the present invention have also proven to be useful in the therapeutic treatment of the dysbiosis of the intestinal microbiota.

Furthermore, the compositions have also turned out to be useful for treatments aimed at reducing halitosis caused by fermentation of protein-containing food residues, effected by proteolytic bacteria.

### Industrial Applicability

The present invention has made it possible to treat and resolve the inflammation at the basis of all diseases that may affect the oral structures. Evident and annoying symptoms such as, for example, reddening, edema, bleeding, spontaneous and induced pain are at least reduced quickly and significantly (in 7/12 days), especially when they show themselves in their chronical forms and in new acute phases. Moreover, topical administration of the compositions of the present invention has also resulted in ameliorated texture (compactness, deep-plane stability and, in some cases, post-inflammatory fibrosis of peri-dental and peri-implant necks) of treated mucosae. In addition, it has also been verified that ingestion of the active principles contained in the compositions of the present invention after topical, oral application thereof can restore a correct balance in the composition of the intestinal flora (eubiosis of the intestinal microbiota). Such a condition, which leads to amelioration of systemic inflammation levels, also affects local inflammation levels (bidirectional action).

## Claims

1. A topical, oral, pharmaceutical composition for use in the treatment of inflammation-based diseases of the oral cavity, wherein the active ingredient consists of at least one probiotic yeast, alive and viable, capable of crossing the gastric barrier undamaged and reaching the gut in alive and viable form, said yeast being the *Kluyveromyces marxianus fragilis* B0399 and being present in an amount ≥ 10⁷ cfu/dose.

2. The composition according to claim 1, for use in the treatment according to claim 1, wherein said at least one alive and viable probiotic yeast is present in an amount ≥ 2•10⁷ cfu/dose.

3. The composition according to any one of the preceding claims, for use in the treatment according to claim 1, further comprising one or more additional active principles or substances performing an adjuvant or complementary function, with the at least one alive and viable probiotic yeast of the preceding claims.

4. The composition according to any one of the preceding claims, for use in the treatment according to claim 1, wherein said inflammation-based diseases of the oral cavity are selected from the group consisting of gingivitis, periodontitis, peri-implant mucositis and peri-implantitis.

5. The composition according to any one of the preceding claims, for use in the treatment according to claim 1, wherein said composition is provided in the form of a swallowable toothpaste.

6. The composition according to any one of the preceding claims, for use in the treatment according to claim 1, wherein said composition is provided in the form of a powder/gel; said powder/gel is inserted into masks custom-made for the dentition/dental arch of the patient, which are applied to the same.

7. The composition according to any one of the preceding claims, for use in the treatment according to claim 1, wherein said composition is provided in the form of a powder/gel; said powder/gel is directly inserted into the patient's gum pockets through a syringe having a suitable needle or cannula.

8. The composition according to any one of the preceding claims, for use in the treatment according to claim 1, wherein said composition is provided in the form of a chewing gum or a chewable or swallowable tablet or a pill or a sweet or another suitable for oral administration.

9. The composition according to any one of the preceding claims, for use in the treatment according to claim 1, and for further simultaneous use in the treatment of the dysbiosis of the intestinal microbiota.

## Patentansprüche

1. Topische, orale, pharmazeutische Zusammensetzung zur Verwendung bei der Behandlung von entzündungsbedingten Erkrankungen der Mundhöhle, wobei der Wirkstoff aus mindestens einer probiotischen Hefe besteht, die lebend und lebensfähig ist und in der Lage ist, die Magenbarriere unbeschädigt zu passieren und den Darm in lebendiger und lebensfähiger Form zu erreichen, wobei es sich bei der Hefe um *Kluyveromyces marxianus fragilis* B0399 handelt und in einer Menge von ≥ 10⁷ cfu/Dosis vorhanden ist.

2. Zusammensetzung nach Anspruch 1 zur Verwendung bei der Behandlung nach Anspruch 1, wobei die mindestens eine lebende und lebensfähige probiotische Hefe in einer Menge ≥ 2-10⁷ cfu/Dosis vorhanden ist.

3. Zusammensetzung nach einem der vorhergehenden Ansprüche zur Verwendung bei der Behandlung nach Anspruch 1, ferner umfassend einen oder mehrere zusätzliche Wirkstoffe oder Substanzen, die eine unterstützende oder ergänzende Funktion zu der mindestens einen lebenden und lebensfähigen probiotischen Hefe der vorhergehenden Ansprüche haben.

4. Zusammensetzung nach einem der vorhergehenden Ansprüche zur Verwendung bei der Behandlung nach Anspruch 1, wobei die entzündungsbedingten Erkrankungen der Mundhöhle aus der Gruppe ausgewählt sind, die aus Gingivitis, Parodontitis, periimplantärer Mukositis und Periimplantitis besteht.

5. Zusammensetzung nach einem der vorhergehenden Ansprüche zur Verwendung bei der Behandlung nach Anspruch 1, wobei die Zusammensetzung in Form einer schluckbaren Zahnpasta bereitgestellt wird.

6. Zusammensetzung nach einem der vorhergehenden Ansprüche zur Verwendung bei der Behandlung nach Anspruch 1, wobei die Zusammensetzung in Form eines Pulvers/Gels bereitgestellt wird; das Pulver/Gel wird in speziell für das Gebiss/den Zahnbogen des/r Patienten/in angefertigte Masken eingefüllt, die auf diese/n aufgetragen werden.

7. Zusammensetzung nach einem der vorhergehenden Ansprüche zur Verwendung bei der Behandlung nach Anspruch 1, wobei die Zusammensetzung in Form eines Pulvers/Gels bereitgestellt wird; das Pulver/Gel wird durch eine Spritze mit einer geeigneten Nadel oder Kanüle direkt in die Zahnfleischtaschen des/r Patienten/in eingeführt.

8. Zusammensetzung nach einem der vorhergehenden Ansprüche zur Verwendung bei der Behandlung nach Anspruch 1, wobei die Zusammensetzung in Form eines Kaugummis oder einer kaubaren oder schluckbaren Tablette oder einer Pille oder eines Bonbons oder einer anderen zur oralen Verabreichung geeigneten Form bereitgestellt wird.

9. Zusammensetzung nach einem der vorhergehenden Ansprüche, zur Verwendung bei der Behandlung nach Anspruch 1 und zur weiteren gleichzeitigen Verwendung bei der Behandlung der Dysbiose der intestinalen Mikrobiota.

## Revendications

1. Composition pharmaceutique topique, orale, destinée à être utilisée dans le traitement de maladies liées à l'inflammation de la cavité buccale, la substance active se composant d'au moins une levure probiotique, vivante et viable, capable de traverser la barrière gastrique intacte et d'atteindre l'intestin sous une forme vivante et viable, ladite levure étant le *Kluyveromyces marxianus fragilis* B0399 et étant présente dans une quantité ≥ 10⁷ ufc/dose.

2. Composition selon la revendication 1, destinée à être utilisée dans le traitement selon la revendication 1, ladite au moins une levure probiotique vivante et viable étant présente dans une quantité ≥ 2•10⁷ ufc/dose.

3. Composition selon l'une quelconque des revendications précédentes, destinée à être utilisée dans le traitement selon la revendication 1, comprenant en outre un ou plusieurs principes actifs ou substances supplémentaires exerçant une fonction adjuvante ou complémentaire, avec ladite levure probiotique vivante et viable des revendications précédentes.

4. Composition selon l'une quelconque des revendications précédentes, destinée à être utilisée dans le traitement selon la revendication 1, lesdites maladies inflammatoires de la cavité buccale étant choisies dans le groupe constitué par la gingivite, la parodontite, la mucosité péri-implantaire et la péri-implantite.

5. Composition selon l'une quelconque des revendications précédentes, destinée à être utilisée dans le traitement selon la revendication 1, ladite composition étant fournie sous la forme d'un dentifrice ingérable.

6. Composition selon l'une quelconque des revendications précédentes, destinée à être utilisée dans le traitement selon la revendication 1, ladite composition étant fournie sous forme de poudre/gel ; ladite poudre/ledit gel est introduit(e) dans des masques fabriqués sur mesure pour la dentition/l'arcade dentaire du patient, qui sont appliqués sur celle-ci.

7. Composition selon l'une quelconque des revendications précédentes, destinée à être utilisée dans le traitement selon la revendication 1, ladite composition étant fournie sous forme de poudre/gel ; ladite poudre/ledit gel est directement introduit(e) dans les poches gingivales du patient à l'aide d'une seringue munie d'une aiguille ou d'une canule appropriée.

8. Composition selon l'une quelconque des revendications précédentes, destinée à être utilisée dans le traitement selon la revendication 1, ladite composition étant fournie sous la forme d'un chewing-gum ou d'un comprimé à mâcher ou à avaler ou d'une pilule ou d'un bonbon ou d'un autre produit adapté à l'administration par voie orale.

9. Composition selon l'une quelconque des revendications précédentes, destinée à être utilisée dans le traitement selon la revendication 1, et destinée à être utilisée simultanément dans le traitement de la dysbiose du microbiote intestinal.
